# EUROPÄISCHE PATENTSCHRIFT

(11) **EP 2 265 721 B1**
(45) Veröffentlichungstag und Bekanntmachung des Hinweises auf die Patenterteilung: **05.10.2016**
(21) Anmeldenummer: 09720455.6
(22) Anmeldetag: 10.02.2009
(51) Int. Cl.: C12P 7/10, D21C 3/20

(54) **Verfahren zur Herstellung von Ethanol aus lignocellulosehaltiger Biomasse**
Process for producing ethanol from lignocellulosic biomass
Procédé de production d'éthanol à partir de biomasse lignocellulosique

(30) Priorität: 12.03.2008 DE 102008013845
(43) Veröffentlichungstag der Anmeldung: 29.12.2010
(73) Patentinhaber: Zylum Beteiligungsgesellschaft mbH & Co. Patente II KG, 12529 Schönfeld/OT Waltersdorf (DE)
(72) Erfinder: KARSTENS, Ties, 79268 Bötzingen (DE)
(74) Vertreter: Patentanwälte und Rechtsanwalt Weiß, Arat & Partner mbB
(86) Internationale Anmeldenummer: PCT/EP2009/000920
(87) Internationale Veröffentlichungsnummer: WO 2009/112134

(56) Entgegenhaltungen:
- WO-A-00/61858
- WO-A-02/29155
- FR-A- 2 518 573
- SHAH, M.M. ET AL.: "Effect of Pretreatment on Simultaneous Saccharification and Fermentation of Hardwood into Acetone/Butanol" APPLIED BIOCHEMISTRY AND BIOTECHNOLOGY, Bd. 28-29, 1991, Seiten 99-110, XP008114715
- BERTRAN, M.S. & DALE, B.E.: "Enzymatic hydrolysis and recrystallization behavior of initially amorphus cellulose", BIOTECHNOLOGY A BIOENGINEERING, vol. 27, no. 2, February 1985 (1985-02), pages 177-181,
- AGARWAL, U.P. ET AL: "AGARWAL, U.P. ET AL.: "ENZYMATIC HYDROLYSIS OF BIOMASS: EFFECTS OF CRYSTALLINITY, PARTICLE SIZE, AND LIGNIN REMOVAL", PROCEEDINGS OF THE 16TH INTERNATIONAL SYMPOSIUM ON WOOD, FIBER AND PULPING CHEMISTRY (ISWFPC), June 2011 (2011-06), pages 910-914,

## Beschreibung

Die vorliegende Erfindung betrifft ein Verfahren zur Herstellung von Bioethanol durch Abtrennen von Lignin aus einer zerkleinerten lignocellulosischen Biomasse und Gewinnung von Cellulose und gegebenenfalls Hemicellulose und Weiterverarbeitung der Cellulose bzw. des Gemisches von Cellulose und Hemicellulose zu Zuckern und anschließend zu Bioethanol.

Lignocellulosische Biomasse ist eine bisher industriell nicht genutzte Quelle von fermentierbaren Zucker. Im Gegensatz zu stärkehaltigen Rohstoffen wie Getreide (Mais, Weizen etc.) ist der enzymatische Abbau der in der lignocellulosischen Biomasse enthaltenen Polysaccharide zu Zuckern allerdings erschwert. Insbesondere liegt der Widerstand der lignocellulosischen Biomasse gegenüber einer enzymatischen Hydrolyse an der kristallinen Phase der Cellulose, an der zugänglichen Oberfläche, an der Ummantelung durch Lignin und schließlich an der Abschirmung der Cellulose durch Hemicellulosen.

Eine Vorbehandlung der Biomasse ist erforderlich, um die Cellulose für Enzyme besser zugänglich zu machen, die die Kohlenhydrate in Zucker überführen sollen. Insofern liegt in der Vorbehandlung der lignocellulosischen Biomasse der Schlüssel für die Verbesserung der Effizienz und die Kostensenkung für cellulosischen Bioethanol. Wichtige Gesichtspunkte für eine wirkungsvolle Vorbehandlung bestehen darin, die Biomasse nicht zu stark mechanisch zerkleinern zu müssen, die Fraktion der Pentosen möglichst zu erhalten, und die Bildung von Nebenprodukten, die die Wirkung der Enzyme vermindern, zu beschränken. Zudem sollte der Energieverbrauch und die Kosten für die Vorbehandlung möglichst gering gehalten werden. Schließlich ist es wünschenswert, das Lignin zu gewinnen und als Nebenprodukt zu vermarkten.

Diese wesentlichen Aspekte für eine industrielle Nutzung von lignocellulosischer Biomasse zur Herstellung von Bioethanol sind zusammengefasst in "features of promising technologies for pretreatment of lignocellulosic biomass" Nathan Mossier et al. Bioresource technology 96 (2005) 673 - 686. D Fengel und G.Wegener kommen in Ihrer Monographie ,,wood, Chemistry, Ultrastructure, Reactions", De Gruyter, Berlin , New York 1989 zu ähnlichen Schlussfolgerungen. Es bestehen zwei hauptsächliche Hindernisse für die Hydrolyse von Cellulose zu Glucose: das Haupthindernis liegt am Lignin, das einen enzymatischen Angriff auf die Cellulose auf ein Minimum herabsetzen kann. Darüber hinaus stellt die Cellulose selbst aufgrund ihrer Kristallinität in der nativen Zellwand einen hohen Widerstand gegenüber einem chemischen und enzymatischen Angriff dar.

Die bekannten Verfahren des Standes der Technik weisen jede für sich spezifische Nachteile auf.

Beim steam explosion-Verfahren werden durch Dampf hoher Temperatur im wesentlichen die Hemicellulosen hydrolysiert, was die Zugänglichkeit für Enzyme verbessert, jedoch aufgrund freigesetzter Säuren zur Inhibierung bei den Enzymen führt. Bei der Behandlung mit heißem Wasser unter Druck bei Temperaturen über 200 °C wird ein großer Teil der Biomasse löslich. Allerdings reagieren die zu Zuckern abgebauten Hemicellulosen weiter zu Aldehyden, wie Furfuralen, die starke Enzyminhibitoren sind.

Die Behandlung mit verdünnter Säure bringt einige schwerwiegende Nachteile mit sich. Infolge der Korrosionsgefahr müssen sehr teure Materialien für die Reaktoren verwendet werden. Die Säure muss durch Kalk neutralisiert werden, bevor die Zucker fermentiert werden. Die damit verbundene Gipsbildung wirkt sich negativ auf die Löslichkeit aus. Die flüssigen Bestandteile müssen vor der enzymatischen Hydrolyse über Ionenaustauscher gereinigt werden. Zudem sinkt die Ausbeute infolge der Bildung von Abbauprodukten und Enzyminhibitoren. Weitere Nachteile stellen die Entsorgung des Gipses, eine lange Enzymbehandlungsdauer und schließlich die sehr energieintensive Mahlung der Biomasse dar.

In der Ammoniakvorbehandlung wird zwar ein hoher Grad an Delignifizierung erzielt, was den Enzymeinsatz zu senken gestattet. Auch der kristalline Charakter der Cellulose wird verändert (das Gitter geht von Cellulose I in Cellulose II über), was die Zugänglichkeit leicht erhöht. Als nachteilig werden allerdings die hohen Kosten für die Ammoniakrückgewinnung angesehen.

Das Dokument SHAH, M.M. ET AL.: "Effect of Pretreatment on Simultaneous Saccharification and Fermentation of Hardwood into Acetone/Butanol" (APPLIED BIOCHEMISTRY AND BIOTECHNOLOGY, Bd. 28-29, 1991, Seiten 99-110) offenbart ein Verfahren zur Herstellung von Ethanol durch Abtrennung von Lignin aus zerkleinertem Espenholz, wobei das zerkleinerte Espenholz mit dem Alkanolamin Monoethanolamin zur Extraktion des darin enthaltenen Lignins behandelt wird, das Lignin extrahiert und die Lösung des Lignins abgetrennt und der anfallende Cellulose/Hemicellulose enthaltende Rückstand ohne Trocknen zu Zuckern umgesetzt wird und die Zucker zu Ethanol fermentiert werden, dadurch gekennzeichnet, dass das zerkleinerte Espenholz direkt mit dem Alkanolamin Monoethanolamin zur Extraktion des darin enthaltenen Lignins behandelt wird. Es erfolgt vor der Extraktion keine Behandlung des zerkleinerten Espenholzes mit einer Lösung von Ammoniak in Ethanolamin.

Das Dokument WO 00/61858 A (RHODIA ACETOW GMBH, 19. Oktober 2000) offenbart ein Verfahren zur Gewinnung von Cellulose und Hemicellulose sowie zur Abtrennung von Lignin aus Holzhackschnitzeln, wobei die vorhydrolysierten Holzhackschnitzel mit dem Alkanolamin Monoethanolamin zur Extraktion des darin enthaltenen Lignins behandelt werden. Vor der Extraktion des Lignins mit Monoethanolamin werden die vorhydrolysierten Holzhackschnitzel mit einer Lösung von Ammoniak in Ethanolamin vorbehandelt. Die gewonnene Cellulose soll zu Cellulosederivaten weiterverarbeitet werden.

Zum Stand der Technik erscheinen noch folgende weitergehende Informationen angebracht:
Ein Nachteil der industriell eingeführten Zellstoffverfahren ist mit Sicherheit der Verlust der Reaktivität infolge von Trocknung. Sogenannter never-dried pulp (nach Aufschluss und Bleiche nicht unter 30% feuchter getrockneter Zellstoff) ist dagegen hochreaktiv. Für die chemische Weiterverarbeitung wäre die nach dem Aufschluss vorliegende Faserform - ungetrocknet - viel besser geeignet.

Bei Steam (Dampf)-Explosion-Prozessen sind nach dem Stand der Technik zur Überschreitung der Erweichungstemperaturen des Lignins und der Polyosen Drücke ab 20 bar üblich. Die mit diesen hohen Drucken verbundenen hohen Dampftemperaturen von über 200°C bedingen einerseits einen starken Kettenabbau der Cellulose, andererseits führen sie zu einer Kondensation des Lignins - dies mit der Folge schichter Extrahierbarkeit. Ein Eindringen des Dampfes in kristalline Bereiche der lignocellulosischen Struktur ist unwahrscheinlich; insofern sind dort Modifizierungen nicht möglich. Der Stand der Technik bei der Steam-Explosion ist in einer Vielzahl von Patenten festgehalten, die sich in erster Linie auf die technische Auslegung und auf die grundsätzlichen Verfahrensweisen beziehen. Die meisten Studien wurden mit Holz als Rohstoff durchgeführt. Der Schwerpunkt lag in der Mehrzahl der Fälle auf der Optimierung des Severity-Faktors (Integral aus dem Produkt von Dampftemperatur und Einwirkzeit).

Ein Nachteil bei der Verwendung von Wasserdampf ist, dass Zusatzstoffe nicht oder nur mit hohem technischen Aufwand im Dampf zugeführt werden können. Vielmehr muss die Biomasse vor der Steam-Explosion mit den einzusetzenden Stoffen in Kontakt gebracht werden, was im allgemeinen mit einer schlechten Verteilung bzw. mit einer höheren Dosierung verknüpft ist. Dies gilt analog auch für den Steam-Refining-Prozeß (Dampfaufschlußverfahren mit anschließender mechanischer Auffaserung), der mit weniger hochgespanntem Dampf arbeitet (10 - 15 bar) und zur Auffaserung mechanische Vorrichtungen verwendet. Das einzige voll kontinuierlich betreibbare Zellstoffaufschlussverfahren, das im technischen Maßstab realisiert ist, stellt die Steam-Explosion von Hackschnitzeln oder anderen Biomassen mit dem Stake System II der Firma StakeTech Stake Technology dar.

Im Zusammenhang mit pulping (Zellstoffaufschluß) sind zwei verschiedene Anwendungen von Ammoniak Stand der Technik: Zerfasern lignocellulosischen Materials unter Verwendung von Ammoniak zum Plastifizieren bei höherer Temperatur durch eine explosionsartige Entspannung (J. J. O'Connor Tappi 55(3), (1972) 353-358) und Plastifizieren von Hackschnitzeln in einem Asplund-Refiner unter Druck mit Ammoniak: das Auffasern des Holzes beanspruchte einen wesentlich geringeren Energieeintrag (R.C. Peterson and R.W. Strauss J. Polymer Sci. C36 (1971) 241-250). In beiden Fällen konnte Lignin nicht weitgehend entfernt werden.

Die Verwendung von Alkanolaminen zur Entfernung von Lignin aus Lignocellulosen wurde erstmals von Elton Fisher und R.S. Bower (J. Am. Chem. Soc. 63 (1941) 1881-1883) beschrieben. In den siebziger Jahren wurde Monoethanolamin als Zusatzstoff zur Natronlauge für den Holzaufschluß bearbeitet (Stichworte: alkaline pulping in aqueous alcohols and amines, acceleration of soda delignification, sulfur free delignification). Damit sollte die Verringerung oder gar der Ersatz schwefelhaltiger Chemikalien ermöglicht werden.

Ein Problem stellte wohl die Aufarbeitung der Aufschlußchemikalien Natronlauge und Ethanolamin und die Abtrennung des Lignins dar. Üblicherweise wird Lignin aus Natronlauge durch Ausfällen unter Säurezugabe gewonnen. Die Ligningewinnung aus der Lösung Natronlauge/Alkanolamin auf diesem Wege hätte die Aufarbeitung und Rückgewinnung der Aufschlußchemikalien sicherlich nicht erleichtert. Die übliche Verbrennung des Lignins in der NaOH nach Eindickung als erstem Schritt der NaOH-Rückgewinnung würde außerdem zum Verlust des Alkanolamins führen, was mit erheblichen Kosten verbunden wäre.

Der Erfindung liegt demnach die Aufgabe zugrunde, ein Verfahren zur Fraktionierung von lignocellulosehaltiger Biomasse bereitzustellen, bei dem die angesprochenen Nachteile des Standes der Technik weitgehend behoben sind. Es sollen die Komponenten Lignin einerseits und Hemicellulose/Cellulose andererseits in möglichst wenig verunreinigter Form getrennt voneinander gewonnen werden, um auf diese Weise Weiterverarbeitern diese Rohstoffe zur Verfügung zu stellen. Es soll hierbei ein schwefel- und chlorfreies. Holzaufschlußverfahren bereitgestellt werden, das außerdem ohne Natronlauge arbeitet und somit auf aufwendige Rückgewinnungs-, Abluft- und Abwasserreinigungsverfahren verzichten kann. Insbesondere soll die Chemiezellstoffgewinnung in zeit-, chemikalien- und energiesparender Weise in kleiner dezentraler Einheit durchgeführt werden können. Die dabei gebildete Cellulose/Hemicellulose soll zunächst in Zucker und darauf in Bioethanol überführt werden. Im Ergebnis soll demzufolge ein besonders vorteilhaftes Verfahren zur Herstellung von Bioethanol durch modifiziertes Verfahren des Standes der Technik zur Gewinnung von reaktiver Cellulose und Hemicellulose aus lignocellulosischer Biomasse bereitgestellt werden.

Erfindungsgemäß wird diese Aufgabe gelöst durch ein Verfahren zur Herstellung von Bioethanol durch Abtrennen von Lignin aus einer zerkleinerten lignocellulosischen Biomasse, wobei die zerkleinerte Biomasse mit einem Alkanolamin, das nicht durch Alkylgruppen am Stickstoff substituiert ist, behandelt wird, das Lignin extrahiert und die Lösung des Lignins abgetrennt und der anfallende Cellulose/Hemicellulose enthaltende Rückstand ohne Trocknen zu Zuckern umgesetzt wird und die Zucker zu Bioethanol fermentiert werden,
dadurch gekennzeichnet, dass die zerkleinerte lignocellulosische Biomasse mit einem Alkanolamin zur Extraktion des darin enthaltenen Lignins behandelt wird, wobei vor der Extraktion eine Behandlung der lignocellulosischen Biomasse mit einer Lösung von Ammoniak in Ethanolamin erfolgt.

Vorteilhafte Ausgestaltungen dieses Verfahrens ergeben sich aus den angeschlossenen Ansprüchen 2 bis 15.

Das erfindungsgemäße Verfahren ermöglicht es, dass Lignocellulosen aufgeschlossen und in ihre Komponenten zerlegt werden und dann der Rückstand mit Alkanolamin einer Extraktion unter Gewinnung eines Celluloserohstoffs (Cellulose/Hemicellulose) unterzogen wird.

Es können alle Arten von Lignocellulose für die erfindungsgemäße Lehre, nämlich die Fraktionierung in die wesentlichen Bestandteile Cellulose, Polyosen und Lignin eingesetzt werden. Als lignocellulosehaltige Biomasse sind Pflanzenwachstumsmaterialien unterschiedlichster Art, wie Holz, Haferhülsen, Mais- und Kornstengel, Bagasse, Stroh aller Art, wie Weizen-, Reis-, Hafer-, Roggen- sowie Maisstroh geeignet. Bei faserigen Rohstoffen, wie Einjahrespflanzen, sind durch Schneiden zerkleinerte Fasern geeignet.

Hier kann eine optionale Behandlung mit Ammoniak folgen. Dies kann an jeder geeigneten Stelle, nach dem angesprochenen Waschschritt, mit wässriger Ammoniaklösung, mit Ammoniakgas oder mit flüssigem Ammoniak durchgeführt werden. Vorzugsweise wird das Massenverhältnis des flüssigen Ammoniaks zu der zu behandelnden Masse (bezogen auf Trockensubstanz) auf etwa 0,1:1 bis 4:1 eingestellt.

Als Alkanolamine kommen alle Alkanolamine in Frage, die nicht durch Alkylgruppen am Stickstoff substituiert sind. So scheiden z.B. aus: N-Methyl-Monoethanolamin und N,N-Dimethyl-Monoethanolamin, da diese keine Wirkung bei der Extraktion des Lignins aus dem Holz zeigen.

Bevorzugt wird als Extraktionsmittel Monoethanolamin eingesetzt, welches in vorgeheizter Form, insbesondere mindestens bei etwa 80°C, eingesetzt werden kann. Es hat sich hierbei gezeigt, dass die Extraktionswirkung von nicht vorbehandelter lignocellulosischer Biomasse zu mit Ammoniak vorbehandelter lignocellulosischer Biomasse eindeutig zunimmt. Der Ligningehalt des Extraktes ist unter gleichen Extraktionsbedingungen bei Ammoniak behandelter Biomasse um ca. 60% höher als bei unbehandelter Biomasse.

Die erfindungsgemäße Extraktion erfolgt vorzugsweise unter Druck, d.h. in einem geeigneten Autoklaven bzw. einem kontinuierlichen Extraktor. Durch eine Extraktion unter Atmosphärendruck können, z.B. bei Stroh, gleich gute Ergebnisse erzielt werden.

Im batch-Betrieb, d.h. in einem Autoklaven, wird die von den Hemicellulosen frei gewaschene, ggf. zerkleinerte und auf geeignete Weise mit Ammoniak vorbehandelte Biomasse mit dem darin enthaltenen Wasser, dem(n) Extraktionsmittel(n), vorzugsweise für mindestens 10 min., insbesondere für mindestens ½ Stunde auf eine Temperatur von etwa 80°C bis 150°C, insbesondere von 100°C bis 140 °C aufgeheizt. Es können dabei auch Lösemittel für die entstehenden Ligninabbaukomponenten bereits zugegen sein.

Bevorzugt gegenüber einer batch-Betriebsweise ist eine kontinuierliche Extraktion. Diese kann ausgeführt werden, indem man die in einen Druckreaktor eingefüllte lignocellulosische Biomasse vom vorgeheizten Extraktionsmittel durchströmen läßt oder indem man das zu extrahierende Gut, die lignocellulosische Biomasse, zum Extraktionsmittel im Gegenstrom führt. Beide Varianten haben gegenüber dem Autoklaven, also dem stationären Betrieb, den Vorteil, dass infolge Abführens der Abbauprodukte mit dem Extraktionsmittel Nebenreaktionen weitgehend ausgeschlossen sind. Zudem kann bei gleicher Extraktionswirkung mit einem geringeren Flottenverhältnis von Extraktionsmittel zu lignocellulosischer Biomasse und bei tieferer Temperatur gearbeitet werden. Die Löslichkeit von Organosolv-Lignin in Monoethanolamin ist hierbei relativ hoch (250 g/Liter).

In einer bevorzugten erfindungsgemäßen Ausführungsform wird die Extraktion mehrstufig, d.h. in mindestens zwei aufeinanderfolgenden Extraktionen mit Alkanolamin durchgeführt. Hierbei wird vorzugsweise die gleiche Gesamtmenge an Alkanolamin wie bei einer einstufigen Extraktion eingesetzt. Hierbei kann in effektiver Weise eine Gegenstromextraktion erfolgen, da hierbei die kürzesten Extraktionszeiten realisiert werden.

Monoethanolamin (im folgenden abgekürzt als: MEA) als Extraktionsmittel weist verschiedene Vorzüge auf. Beim Aufschluß verhindert MEA die Ligninkondensation und das Aufpfropfen auf Cellulose, es schützt die Cellulose vor DP-Abbau und verbessert die Delignifzierung.

Die Alkanolamin-Extraktion kann bei niedrigeren Temperaturen (etwa 100 bis 120°C) durchgeführt werden, insbesondere wenn eine Ammoniak-Vorbehandlung vorgenommen wird. Trotz der niedrigen Temperaturen werden dann niedrige kappa-Zahlen erhalten. Außerdem werden Nebenreaktionen stark zurückgedrängt.

Nach der Extraktionsstufe wird der Celluloserohstoff (Cellulose/Hemicellulose) gewonnen. Hierzu wird der stark dunkelbraun bis schwarz gefärbte Ligninextrakt von den Rohzellstofffasern auf geeignete Weise nach den für die fest/flüssig-Trennung üblichen Verfahren abgetrennt. Ist ein vollständiges Entfernen der im Celluloserohstoff noch verbleibenden Reste an prozessmodifiziertem Lignin erwünscht, kann dieses mit einem geeigneten Lösemittel durch Wäsche oder Gegenstromwäsche extrahiert werden. Das hierbei verwendete Lösemittel wird anschließend durch Destillation vom Lignin und vom Extraktionsmittel abgetrennt, auf diese Weise zurückgewonnen und steht damit wieder zur Verfügung.

Der Rückstand nach dem Abdestillieren des Lösemittels kann zudem mit dem von den Fasern abgetrennten Extrakt vereinigt werden. Daraus werden Wasser und das als Extraktionsmittel dienende Alkanolamin durch Destillation, vorzugsweise Vakuumdestillation abgetrennt. Auch andere Trennprozesse, die wunschgemäß zum Einengen des Ligninextraktes - im Grenzfall bis zur Trockenmasse - führen, sind hier geeignet. Eine Abtrennung des Lignins gelingt auch, indem ein Nichtlösemittel zu der Lösung des Lignins in Alkanolamin zugegeben wird. Dabei fällt das Lignin in Form von Feststoffpartikeln aus und kann durch einen geeigneten Fest/Flüssigtrennprozeß, wie Filtration, Zentrifugation, Dünnschichtverdampfung oder Membrantrennverfahren vom Extraktionsmittel Alkanolamin abgetrennt werden. Die Abtrennung des Lignins kann zum Beispiel durch Einleiten von CO₂ in den mit Wasser oder besser mit dem Waschwasser nach der Alkanolamin-Extraktion verdünnten, gegebenenfalls aufkonzentrierten Lignin/Alkanolamin-Extrakt erfolgen. Durch das Aufkonzentrieren mittels einer Dünnschichtverdampfung oder einer geeigneten anderen Destillationsmethode wird ein Großteil des Alkanolamins in reiner Form zurückgewonnen. Der Rest des Alkanolamins wird destilliert, nachdem das Wasser aus der Fällflüssigkeit nach Abtrennen des Lignins - ebenfalls im Vakuum - abdestilliert wurde. Die Ligninausfällung gelingt somit durch Einleiten von CO₂ und Abzentrifugieren. Die sich mit dem CO₂ bildende Additionsverbindung Alkanolamin*CO₂ kann thermisch bzw. durch Eindüsen von Dampf wieder vollständig in Alkanolamin und CO₂ zersetzt werden. Der Rückstand besteht aus in seiner Molmasse stark abgebautem, chemisch nicht wesentlich verändertem Lignin. Dieses kann daher als chemischer Rohstoff, z.B. für die Herstellung von Duroplasten oder von Polyurethanen, weiterverwendet werden.

Der Rohzellstoff weist eine kappa-Zahl von höchstens etwa 20, vorzugsweise unter etwa 10 auf. Dies entspricht einem Ligningehalt von <3 bzw. <1,5 Masse-% und stellt einen vorteilhaften Einstieg in die enzymatische Verzuckerung des Rohzellstoffs dar.

In der Praxis kann ein sogenannter "inclined screw reactor" verwendet werden. Bevorzugt ist zum Beispiel die Verwendung eines "inclined screw reactors" für die Vorbehandlung mit NH₄OH/Alkanolamin, dann eine oben genannte Auffaserung für die gründliche Ligninextraktion in Form der Gegenstromextraktion. Diese beiden Schritte ergeben im ersten Fall eine schwach ligninhaltige, wasserreiche Fraktion, die mehrfach eingesetzt werden kann, wobei das Lignin aufkonzentriert wird, und im zweiten Fall eine stark ligninhaltige und Alkanolamin-reiche Fraktion. Die Vereinigung der beiden Fraktionen im Verhältnis wasserreich/Alkanolamin-reich ∼2/1 ermöglicht das Ausfällen des Lignins durch CO₂ bei erhöhter Temperatur. Aus der Alkanolamin-reichen und wasserarmen Fraktion braucht nur wenig Wasser abdestilliert zu werden, um dann beispielsweise durch eine Dünnschichtverdampfung den größten Teil an Alkanolamin zurückgewinnen zu können und dabei gleichzeitig die Ligninkonzentration um mehr als 20% zu erhöhen.

Mit der Erfindung ist eine Vielzahl von Vorteilen verbunden:

Ein Vorteil liegt zunächst darin, dass keinerlei schwefelhaltige Chemikalien für den Aufschluß verwendet werden.

Setzt man Getreidestroh, wie vorstehend bereits angesprochen, als Ausgangsmaterial ein, so entfällt die bei NaOH mehrstufige und aufwendige klassische Rückgewinnung oder die Nassoxidation. Bei Alkanolamin besteht die Rückgewinnung in einer einfachen Vakuumdestillation. Die sich mit dem CO₂ bildende Additionsverbindung Alkanolamin*CO₂ kann thermisch bzw. durch Eindüsen von Dampf wieder vollständig in Alkanolamin und CO₂ zersetzt werden. Die Notwendigkeit der Rückgewinnung von Natriumsalzen entfällt. Die Alkanolamin-Extraktion kann bei dem geringen Wassergehalt des Strohs auch drucklos durchgeführt werden, was die Verwendung von einfachen Apparaten gestattet.

Darüber hinaus erlaubt die Erfindung die Extraktion mit einem Alkanolamin bei wesentlich geringeren Flottenverhältnissen (etwa 3:1) - insbesondere beim kontinuierlichen Betrieb. Dies wirkt sich positiv auf den Dampfverbrauch bei Extraktion und Rückgewinnung aus (etwa 3:1 bis 1:1), als bei klassischen Aufschlussverfahren.

Kleine dezentrale Einheiten zur Produktion von Rohzellstoff können unter optimalen wirtschaftlichen Bedingungen betrieben werden, wenn eine Rückgewinnung des Extraktionsmittels Alkanolamin erfolgen sollte. Die Verwendung von Alkanolaminen als Extraktionsmittel ist demnach in zweifacher Hinsicht vorteilhaft: die Rückgewinnung durch Destillation ist bei den diesen Stoffen eigenen Verdampfungswärmen nicht energieaufwendig. Die Abtrennung des Lignins kann ohne Verwendung von Säuren durchgeführt werden, was ebenfalls aufwendige Aufarbeitungsprozesse vermeidet und die Umwelt schont.

Zusatzstoffe zur Verhinderung der Kondensation des Lignins sind auch nicht erforderlich, was Kosten spart. Da eine Vorbehandlung mit Chemikalien, wie sie bei ausschließlichem Einsatz des Steam-Explosion- und des Steam-Refining-Verfahrens nötig ist, entfallen kann, gibt es keine Probleme mit deren Verteilung.

Zudem weisen die Rohzellstoffe nach der Extraktion mit Alkanolamin eine gegenüber anderen Prozessen erhöhte Dekristallisation auf. Dies ist zweifelsohne für die Weiterverarbeitung zu Bioethanol sehr günstig, da hiermit die gleichmäßige Zugänglichkeit für Enzyme zur Herstellung der Zucker gegeben ist. Die Möglichkeit der Verwendung von Ammoniak in weitaus geringerer Menge als bei der reinen Ammoniakexplosion von Biomasse wirkt sich ebenfalls günstig auf die Betriebskosten aus. Ein aufwendiges Rückgewinnen von flüssigem Ammoniak kann entfallen.

Da der zeitliche Aufwand für die Saccharifizierung naturgemäß umso geringer ist, je niedriger die kappa-Zahlen (die Ligningehalte) nach der Extraktion sind, liegen im Verfahren der Erfindung ebenfalls günstige Voraussetzungen vor. Infolge eines vollkontinuierlichen Betriebes liegen die spezifischen Investitionen demzufolge niedrig und die Raum-/Zeitausbeuten sind hoch. Es können einfache, kommerziell erhältliche Anlagenkomponenten verwendet werden. Dies erlaubt einen wirtschaftlichen Betrieb schon mit kleinen Anlagengrößen. Es kann ferner eine Kreislaufschließung bei der Alkanolamin-Rückgewinnung erfolgen. Hierdurch liegt ein geringerer Aufwand beim Aufkonzentrieren, weniger Wasserverbrauch und weniger Energiekosten bei der Destillation vor.

Wird im erfindungsgemäßen Verfahren eine mehrstufige Alkanolamin-Extraktion oder eine kontinuierliche anstelle einer einstufigen gewählt, so bieten diese Alternativen hinsichtlich Effizienz und Leistungsfähigkeit der Verfahrensführung weitere Vorteile.

Eine kontinuierliche Prozeßführung hat zudem Vorteile: Es entfällt die Verdrängung einer Prozeßflüssigkeit durch eine andere, wie dies bei der klassischen pulping-Technologie in Kochern nötig ist. In Kochern sind die Austausch- und Waschprozesse weniger effektiv und dauern dabei relativ lang. Zudem ist die Trennschärfe bei der Verdrängung in der Praxis so, dass es zu unerwünschten Vermischungen oder zu unscharfen Übergängen kommt, sodass zusätzlicher Aufwand bei der Rückgewinnung erforderlich wird. Bei einem kontinuierlichen Verfahren wird der Einsatz von Apparaten möglich, die nicht auf hohe Drucke ausgelegt sein müssen.

Wie oben dargestellt, wird erfindungsgemäß eine lignocellulosische Biomasse in vorteilhafter Weise zu Cellulose/Hemicellulose mit einer besonders günstigen Reaktionsfähigkeit weiterverarbeitet. Im Rahmen der Erfindung wird dieses Verfahrenserzeugnis in fachmännischer Weise zu Zuckern umgewandelt und diese werden zu Bioethanol fermentiert. Dieses Fachwissen soll nachfolgend dargestellt werden:

Der Abbau von cellulosischen Substanzen, hier im Rahmen der Erfindung von Lignin befreite Cellulose/Hemicellulose, erfolgt durch Cellulasen. Diese Bezeichnung bezieht sich in jedem Fall eher auf eine Mischung von Enzymen, denn auf ein einzelnes. Cellulasen werden von Mikroben wie Bakterien und Pilzen produziert. Als ein Beispiel sei genannt Trichoderma. Die hauptsächlichen Bestandteile von Cellulasen sind β-1,4-Gluconasen, die die Primärreaktion der hydrolytischen Spaltung der β-1,4-glycosidischen Bindungen zwischen den Anhydroglucoseeinheiten in der Cellulosekette bewirken.

Es gibt zwei Arten von hydrolytisch wirkenden Enzymen, nämlich 1) die Endo- enzyme (endo-ß-1,4-Gluconasen), die die Spaltung von glykosidischen Bindungen an verschiedenen Stellen der Cellulosemoleküle unter Bildung von oligomeren Mischungen von Kettenbruchstücken hervorrufen, und 2) die exo-Enzyme (exo-ß-1,4-Gluconasen), die glykosidische Bindungen in den Cellulosemolekülen an Stellen in Nachbarschaft zu den nicht reduzierenden Kettenenden spalten, wobei niedermolekulare Fragmente, hauptsächlich Cellobiose oder Glucose, entstehen. Daneben gibt es oxidativ wirksame Enzymsysteme, im allgemeinen Gluco-Oxidasen genannt. Deren Rolle im enzymatischen Abbau ist noch in Diskussion. Weiterführende begriffliche Klärungen und eine summarische Klassifizierung der im enzymatischen Abbau von Cellulose beteiligten Enzyme sind von P. Finch and J.C. Roberts (1985) in "Enzymatic degradation of cellulose" in : Cellulose Chemistry and its Application (Eds: Nevell, T.P., and Zeronian, S.H.), Chapter 13, p. 312) Ellis Horwood Ltd, Chichester dargestellt worden.

Die Vergärung bzw. Fermentierung der Zucker, insbesondere Hexosenzucker, wird im allgemeinen mit der Hefe Saccharomyces cerevisiae durchgeführt, diejenige von Pentosen (xylose) mit dem Pilz Fusarium spec.. Bei gemischten Kulturen für Hydrolyse und Fermentation von Xylose werden zwei anaerobe thermophile Bakterien, nämlich Clostridium thermocellum und Thermoanaerobacter ethanolicus, in Gegenwart von celukosischem Material eingesetzt. In der industriellen enzymatischen Hydrolyse werden die Enzyme, z.B. Z. mobilis, an Ort und Stelle produziert. Vorzugsweise werden die enzymatische Hydrolyse und die Fermentation in einem Schritt, der sog. simultaneous saccharification and fermentation (SSF), durchgeführt.

Immer gilt jedoch in diesem Zusammenhang, dass eine wirkungsvolle Vorbehandlung, wie die Ammoniak in MEA Vorbehandlung, einen wesentlichen Schritt für die Wirksamkeit und die Dauer der enzymatischen Hydrolyse darstellt.

Bei dem Auffinden der vorliegenden Erfindung erlangten die Erfinder verschiedene Erkenntnisse, die in ihrer Darstellung das Verständnis vorliegender Erfindung erleichtern. Hierzu im Einzelnen:

Die Vorbehandlung mit Alkanolamin, insbesondere Monoethanolamin (MEA), von lignocellulosischer Biomasse kennt viele der geschilderten Nachteile des Standes der Technik nicht. Monoethanolamin ist in der Lage, spezifisch Lignin abzubauen und zu lösen. Dagegen übt es auf Polysaccharide, d.h. Cellulose/Hemicellulose, eine schützende Wirkung aus, da es nicht wie andere Alkalien (z.B. Natronlauge) die Polysaccharidketten abbaut (peeling reaction). Neben der vollständigen Entfernung des Lignins hat MEA eine quellende Wirkung auf Cellulose und Hemicellulosen. So werden etwa der Fibrillationsgrad und die Kristallitgrössen verringert, wodurch größere Zwischenräume und ein höherer Anteil an zugänglicher Oberfläche erzeugt werden. Dies ist durch Messungen des Wasser - und Essigsäurerückhaltevermögens nachgewiesen worden. Der Anteil an amorphen Strukturen wird vergrößert, was durch Röntgenbeugung nachgewiesen wurde.

Das Lösemittelrückhaltevermögen als Maß für die Zugänglichkeit beträgt für MEA 108%, für Wasser 51%. Amine können bekanntlich cellulosische Substrate dekristallisieren. Eine Ethylaminbehandlung hebt den Anteil an ungeordneter (dekristallisierter) Cellulose von 9% auf 70%. Ein wichtiger Faktor für die dekristallisierende Wirkung ist der sog. pKa- Wert, der ein Mass für die Basizität ist. Diese wiederum ist von Bedeutung für das Aufbrechen der Wasserstoffbrückenbindungen. MEA hat bei Konzentrationen von mehr als 50% in Wasser einen pH - Wert von 12,5. Nach einer MEA - Vorbehandlung kann durch Wäsche mit heissem Wasser das in MEA gelöste Lignin quasi quantitativ entfernt werden. Dabei wird die durch die MEA - Vorbehandlung hervorgerufene Quellung der Cellulose und Hemicellulosen beibehalten. Die Quellung erfolgt intrafibrillär, d.h. in den kristallinen Cellulosefibrillen, als auch interfibrillär. Ein Beispiel für effektive intrakristalline Quellung ist die Behandlung von Cellulose mit flüssigem Ammoniak. Ammoniak dringt in die kristallinen Bereiche ein, wodurch der Abstand zwischen den 101 - Ebenen des Kristallgitters aufgeweitet wird.

Der Grad an Zugänglichkeit kann auch mittels Jodabsorption durch die Proben ermittelt werden, die auf unterschiedliche Weise vorbehandelt wurden. Die Größe der Jodmoleküle beschränkt deren Absorption auf die amorphen Bereiche der Probe. Auf diese Weise ist der Grad der Jodabsorption direkt dem Anteil an amorphen Bereichen proportional. Unbehandelte Cellulose absorbiert typischerweise ∼ 50 mg Jod / g Probe, was in etwa 15 % Anteil an amorpher Phase entspricht (ermittelt aus Röntgenbeugungsmessungen der Kristallinität der gleichen Probe). Eine mit gasförmigem Ammoniak behandelte, eine mit flüssigem Ammoniak gequollene und schließlich eine mit MEA delignifizierte Biomasse Probe weisen 125, 160 bzw. 230 mg absorbiertes Jod / g Probe auf. Demnach ist die Zugänglichkeit für Enzyme bei der mit MEA delignifizierten Probe ca. 4,6 mal grösser als bei unbehandeltem Zellstoff bzw. der Anteil an leicht zugänglichen amorphen Bereichen beträgt 15*4,6 ∼ 70%.

Das Wasserstoffbrückenbindungsvermögen der Spezies, die mit der Cellulose in Wechselwirkung treten, spielt eine herausragende Rolle. Besonders hohe Quellung kann durch Flüssigkeiten hervorgerufen werden, die nicht-assoziierte Protonenakzeptoren sind. Beispiele dafür sind Ammoniak (pKa = 9,25), Methylamin (pKa=10,69), Ethylamin (pKa= 10,81) und Monoethanolamin (pKa= 9,5). Somit verfügt MEA über einen pKa - Wert nahe dem des Ammoniaks.

Lösungen von Ammoniak in Ethanolamin vermögen Cellulose nicht nur unter Strukturveränderungen zu quellen, sondern das durch Ammoniak aufgeweitete Gitter der Cellulose noch weiter aufzuweiten. So ist das Wasserrückhaltevermögen (WRV) von Cellulose in mit einem Anteil von 10% Ammoniak in Wasser maximal 50%. Dagegen erfolgt eine Zunahme des WRV mit 10% Ammoniak in MEA auf 115%. Es ist daraus zu schliessen, dass MEA das Eindringen des Ammoniaks in das Cellulosegitter begünstigt und das durch Ammoniak aufgeweitete Gitter der Cellulose noch weiter aufweitet. Dies lässt den Schluss zu, dass geringe Mengen an Ammoniak in MEA vorteilhaft sind. Dies betrifft nicht nur die erhöhte Enzymzugänglichkeit, sondern auch die neutralisierende Wirkung des Ammoniaks auf in den Lignocellulosen gebundene Säuregruppen (Acetyl-, Uron- und Formylreste).

Die Vorteile der erfindungsgemäßen Behandlung von lignocellulosischer Biomasse sind daher vielfältig. Vermieden werden kann die Abtrennung von Salzen, da keine Säure eingesetzt wird. Es müssen Enzyminhibitoren vor der enzymatischen Hydrolyse nicht durch Ionenaustausch entfernt werden. Mehr als 95% des Lignins werden aus der Biomasse entfernt. Dieses Lignin ist ein Organosolvlignin, ähnlich dem aus dem Acellverfahren gewinnbaren Lignin, und kann entweder für die Dampf- und anschliessende Stromerzeugung verwendet werden. Aufgrund der teerartigen Konsistenz des vom MEA durch Destillation befreiten Lignins sind für die Dampferzeugung keine kostspieligen Biomasse- Boiler erforderlich. Alternativ kann das Lignin ausgefällt werden und dann zur Herstellung von Polymeren, Langzeitdünger etc. verkauft werden. Für die Ausfällung des Lignins kann vorteilhaft das bei der Vergärung der Zucker zu Ethanol in grosser Menge entstehende Kohlendioxid verwendet werden. Insofern wird durch das Ammoniak in MEA - Vorbehandlungsverfahren eine Forderung erfüllt, nämlich ein höher-wertiges Nebenprodukt zu erzeugen. Die Abtrennung des Lignins bietet gegenüber anderen Verfahren den Vorteil einer höheren Enzymreaktionsgeschwindigkeit um den Faktor 3 - 5.

Zudem können durch die Abtrennung des Ligninanteils der Biomasse, der ca. 18 - 25 Masse -% beträgt, die Apparategrössen entsprechend kleiner ausgelegt werden. Dazu kommt, dass nach der Wäsche zur Entfernung des Lignins und des Monoethanolamins der Feststoffanteil der delignifizierten Fasern ca. 40- 45 % beträgt, was ebenfalls zu einer Reduzierung der Enzymreaktorkapazität beitragen kann. Dieser Faktor wirkt sich entscheidend sehr positiv auf die Herstellkosten des Bioethanols aus.

Schliesslich kann festgestellt werden, dass das erfindungsgemäße Ammoniak in MEA - Vorbehandlungsverfahren die Forderung "low energy, high yield, nodetox" erfüllt, die bei der industriellen Produktion von Bioethanol aus lignocellulosischer Biomasse eine Maxime darstellt.

### Beispiel 1 (nicht erfindungsgemäß)

Eine mit Ethanolamin behandelte und anschließend mit heissem Wasser von Lignin und Ethanolamin befreite Biomasse wird in eine Flasche, die 50 ml Zitratpuffer (pH-Wert 4,5) enthält, zugegeben, so dass die Konzentration 2 Masse-% beträgt. Die Flasche wurde bei 120°C autoklaviert. Es werden 2 ml Cellulase (3 - 4 IU / g vorbehandelter Biomasse) zugegeben. Die Hydrolyse wird bei einer Temperatur von konstant 50°C in einem Wasserbad unter Schütteln der Flasche durchgeführt. Die Zucker werden durch HPLC analysiert. Nach 36 Stunden erreicht die Umsetzung zu Glucose und Xylose einen konstanten Wert. Die Ausbeuten für Glucose und Xylose betragen 90% bzw. 85%. Anschließend wird das erhaltene Gemisch einer üblichen Fermentierung unterzogen.

Für die Fermentation wurde das Bakterium Z. mobilis unter Zusatz von Diammoniumphosphat verwendet. Dieser Biokatalysator wandelt Glucose und Xylose in Ethanol um. Die Ausbeute an Ethanol aus der Glucose betrug 90%, diejenige aus Xylose 85%.

## Patentansprüche

1. Verfahren zur Herstellung von Bioethanol durch Abtrennen von Lignin aus einer zerkleinerten lignocellulosischen Biomasse, wobei die zerkleinerte lignocellulosische Biomasse mit einem Alkanolamin, das nicht durch Alkylgruppen am Stickstoff substituiert ist, behandelt wird, das Lignin extrahiert und die Lösung des Lignins abgetrennt und der anfallende Cellulose/Hemicellulose enthaltene Rückstand ohne Trocknen zu Zuckern umgesetzt wird und die Zucker zu Bioethanol fermentiert werden, **dadurch gekennzeichnet, dass** die zerkleinerte lignocellulosische Biomasse mit dem Alkanolamin zur Extraktion des darin enthaltenen Lignins behandelt wird, wobei vor der Extraktion eine Behandlung der lignocellulosischen Biomasse mit einer Lösung von Ammoniak in Ethanolamin erfolgt.

2. Verfahren nach Anspruch 1, **dadurch gekennzeichnet, dass** als lignocellulosische Biomasse Getreidestroh, Gräser, Bagasse, Pappel und Bambus eingesetzt werden.

3. Verfahren nach Anspruch 1 oder 2, **dadurch gekennzeichnet, dass** bei der Extraktion des Lignins zusätzlich ein weiteres Lösungsmittel für Lignin herangezogen wird.

4. Verfahren nach einem der Ansprüche 1 bis 3, **dadurch gekennzeichnet, dass** zur Vorbehandlung mit Ammoniak Ammoniakgas oder wässrige Ammoniaklösung herangezogen werden.

5. Verfahren nach einem der Ansprüche 1 bis 3, **dadurch gekennzeichnet, dass** die Extraktion mindestens eine halbe Stunde lang bei einer Temperatur von 80°C bis 150°C durchgeführt wird.

6. Verfahren nach einem der Ansprüche 1 bis 4, **dadurch gekennzeichnet, dass** das Alkanolamin vor der Extraktion auf eine Temperatur von mindestens etwa 80°C erwärmt wird.

7. Verfahren nach mindestens einem der vorhergehenden Ansprüche, **dadurch gekennzeichnet, dass** als Alkanolamin Monoethanolamin eingesetzt wird.

8. Verfahren nach mindestens einem der vorhergehenden Ansprüche, **dadurch gekennzeichnet, dass** nach der Extraktion des Lignins am Rückstand haftende Lösung des Ligninrestes durch ein weiteres Lösungsmittel entfernt wird.

9. Verfahren nach Anspruch 8, **dadurch gekennzeichnet, dass** nach der Extraktion am Rückstand haftende Lösung des Ligninrestes durch Wäsche oder Gegenstromwäsche mit dem weiteren Lösungsmittel entfernt wird, anschließend das weitere Lösungsmittel sowie das Extraktionsmittel Alkanolamin durch Destillation vom Lignin abgetrennt und das Alkanolamin für einen erneuten Einsatz rückgewonnen wird.

10. Verfahren nach mindestens einem der Ansprüche 1 bis 7, **dadurch gekennzeichnet, dass** nach der Extraktion am Rückstand haftende Lösung des Ligninrestes davon durch Abpressen oder Abschleudern entfernt wird.

11. Verfahren nach mindestens einem der Ansprüche 1 bis 6, **dadurch gekennzeichnet, dass** das im Alkanolamin gelöste Lignin durch Zugabe eines Nichtlösemittels ausgefällt und durch Fest-/Flüssigtrennprozesse abgetrennt wird.

12. Verfahren nach Anspruch 11, **dadurch gekennzeichnet, dass** das ausgefällte Lignin durch Filtrieren oder Zentrifugieren vom Alkanolamin abgetrennt wird.

13. Verfahren nach mindestens einem der Ansprüche 1 bis 6, **dadurch gekennzeichnet, dass** das im Alkanolamin gelöste Lignin in einem Dünnschichtverdampfer oder durch einen Membranprozess abgetrennt wird.

14. Verfahren nach mindestens einem der vorhergehenden Ansprüche, **dadurch gekennzeichnet, dass** das Cellulose und gegebenenfalls Hemicellulose enthaltene feuchte Zwischenerzeugnis durch enzymatische Hydrolyse zu Zuckern umgesetzt wird.

15. Verfahren nach mindestens einem der vorhergehenden Ansprüche, **dadurch gekennzeichnet, dass** die aus Cellulose und gegebenenfalls aus Hemicellulosen gewonnenen Zucker dadurch zu Bioalkohol fermentiert werden, indem man diese mit Hefen versetzt und vergärt.

## Claims

1. A method of producing bioethanol by separating lignin from a comminuted lignocellulosic biomass, wherein the comminuted lignocellulosic biomass is treated with an alkanolamine that is not substituted by alkyl groups on the nitrogen atom, the lignin is extracted and the lignin solution separated, and the cellulose-/hemicellulose-containing residue obtained is converted to sugars without drying and the sugars are fermented to obtain bioethanol, **characterised in that** the comminuted lignocellulosic biomass is treated with the alkanolamine to extract the lignin contained therein, a treatment of the lignocellulosic biomass with a solution of ammonia in ethanolamine taking place prior to the extraction.

2. The method according to claim 1, **characterised in that** cereal straw, grasses, bagasse, poplar and bamboo are employed as lignocellulosic biomass.

3. The method according to claim 1 or 2, **characterised in that** during extraction of the lignin an additional solvent for lignin is used.

4. The method according to one of claims 1 to 3, **characterised in that** ammonia gas or aqueous ammonia solution is used for the pretreatment with ammonia.

5. The method according to one of claims 1 to 3, **characterised in that** the extraction is performed for at least half an hour at a temperature of 80°C to 150°C.

6. The method according to one of claims 1 to 4, **characterised in that** the alkanolamine is heated to a temperature of at least about 80°C before the extraction.

7. The method according to at least one of the preceding claims, **characterised in that** monoethanolamine is employed as the alkanolamine.

8. The method according to at least one of the preceding claims, **characterised in that** solution of the remainder of the lignin adhering to the residue after lignin extraction is removed by an additional solvent.

9. The method according to claim 8, **characterised in that** solution of the remainder of the lignin adhering to the residue after extraction is removed by washing or countercurrent washing with the additional solvent, after which the additional solvent and the extraction agent alkanolamine are separated from the lignin by distillation and the alkanolamine is recovered to be used again.

10. The method according to at least one of claims 1 to 7, **characterised in that** solution of the remainder of the lignin adhering to the residue after extraction is removed therefrom by squeezing or centrifuging.

11. The method according to at least one of claims 1 to 6, **characterised in that** the lignin dissolved in the alkanolamine is precipitated by adding a non-solvent and separated by solid/liquid separation processes.

12. The method according to claim 11, **characterised in that** the precipitated lignin is separated from the alkanolamine by filtering or centrifuging.

13. The method according to one of claims 1 to 6, **characterised in that** the lignin dissolved in the alkanolamine is separated in a thin-film evaporator or by a membrane process.

14. The method according to at least one of the preceding claims, **characterised in that** the moist intermediate product containing the cellulose and possibly hemicellulose is converted to sugars by enzymatic hydrolysis.

15. The method according to at least one of the preceding claims, **characterised in that** the sugars obtained from cellulose and possibly from hemicelluloses are fermented to form bioalcohol by adding and fermenting with yeasts.

## Revendications

1. Procédé de production de bioéthanol par séparation de lignine d'une biomasse lignocellulosique broyée, dans lequel la biomasse lignocellulosique broyée est traitée avec une alcanolamine qui n'est pas substituée par des groupes alkyle sur l'azote, la lignine est extraite et la solution de la lignine est séparée et le résidu contenu dans la cellulose/hémicellulose résultant est converti sans séchage en sucres et les sucres sont fermentés pour obtenir de l'éthanol, **caractérisé par le fait que** la biomasse lignocellulosique broyée est traitée avec l'alcanolamine pour l'extraction de la lignine y contenue, avant l'extraction ayant lieu un traitement de la biomasse lignocellulosique avec une solution d'ammoniac dans de l'éthanolamine.

2. Procédé selon la revendication 1, **caractérisé par le fait que** comme biomasse lignocellulosique sont utilisés de la paille de céréales, des herbes, de la bagasse, du peuplier et du bambou.

3. Procédé selon la revendication 1 ou 2, **caractérisé par le fait que** lors de l'extraction de la lignine est utilisé en plus un autre solvant pour la lignine.

4. Procédé selon l'une des revendications 1 à 3, **caractérisé par le fait qu'**est utilisé, pour le prétraitement avec de l'ammoniac, de l'ammoniac gazeux ou une solution aqueuse d'ammoniac.

5. Procédé selon l'une des revendications 1 à 3, **caractérisé par le fait que** l'extraction est effectuée pendant au moins une demi-heure à une température de 80°C à 150°C.

6. Procédé selon l'une des revendications 1 à 4, **caractérisé par le fait que** l'alcanolamine est, avant l'extraction, chauffée à une température d'au moins environ 80°C.

7. Procédé selon au moins l'une des revendications précédentes, **caractérisé par le fait que** comme alcanolamine est utilisée une monoéthanolamine.

8. Procédé selon au moins l'une des revendications précédentes, **caractérisé par le fait que**, après l'extraction de la lignine, la solution du reste de lignine adhérant au résidu est éliminée par un autre solvant.

9. Procédé selon la revendication 8, **caractérisé par le fait que**, après l'extraction, la solution du reste de lignine adhérant au résidu est éliminée par lavage ou par lavage à contre-courant avec l'autre solvant, ensuite l'autre solvant ainsi que l'agent d'extraction alcanolamine sont séparés par distillation de la lignine et l'alcanolamine est récupérée pour une nouvelle utilisation.

10. Procédé selon au moins l'une des revendications 1 à 7, **caractérisé par le fait que**, après l'extraction, la solution du reste de lignine adhérant au résidu est éliminée par pressage ou centrifugation.

11. Procédé selon au moins l'une des revendications 1 à 6, **caractérisé par le fait que** la lignine dissoute dans l'alcanolamine est précipitée par addition d'un non-solvant et séparée par des procédés de séparation de solides/liquides.

12. Procédé selon la revendication 11, **caractérisé par le fait que** la lignine précipitée est séparée de l'alcanolamine par filtration ou centrifugation.

13. Procédé selon au moins l'une des revendications 1 à 6, **caractérisé par le fait que** la lignine dissoute dans l'alcanolamine est séparée dans un évaporateur à couche mince ou par un procédé à membrane.

14. Procédé selon au moins l'une des revendications précédentes, **caractérisé par le fait que** le produit intermédiaire humide contenu dans la cellulose et éventuellement l'hémicellulose est converti en sucres par hydrolyse enzymatique.

15. Procédé selon au moins l'une des revendications précédentes, **caractérisé par le fait que** les sucres obtenus à partir de la cellulose et éventuellement des hémicelluloses sont fermentés pour obtenir du bio-alcool en les mélangeant et fermentant avec de la levure.
